# EUROPEAN PATENT APPLICATION

(11) **EP 1 892 671 A2**
(43) Date of publication of application: **27.02.2008**
(21) Application number: 07015666.6
(22) Date of filing: 09.08.2007
(51) Int. Cl.: G06T 7/60, A61B 8/00

(54) **System and method for determining the volume of an object by image processing**

(30) Priority: 23.08.2006 KR 20060080015
(71) Applicant: MEDISON CO., LTD., Kangwon-do 250-870 (KR)
(72) Inventor: Choi, Doo Hyun, Gangnam-gu Seoul 135-280 (KR); Kwon, Eui Chul, Gangnam-gu Seoul 135-280 (KR); Kim, Sung Yun, Gangnam-gu Seoul 135-280 (KR)
(74) Representative: Lorenz, Markus

(57) **Abstract**

Embodiments of the present invention may provide an image processing system and method for calculating the volume of a specific portion in a targeted body. According to an embodiment of the present invention, an image processing system may comprise a volume data receiver, a user interface, a display and a main processor. The volume data receiver may form volume data of a target object based on image data thereof. The user interface may receive reference section information on a reference section of the target object, slice section information on a plurality of slice sections each being perpendicular to the reference section and contour information from a user. The display may form an image of the reference section and images of the slice sections based on the volume data. The main processor may set a reference sectional object and slice sectional objects based on the reference section information and the slice section information, respectively. The main processor may be further operable to (i) detect a contour of the reference sectional object based on the contour information, (ii) to detect seed points of the target object in the images of the slice sections based on the contour information and the slice section information, (iii) to detect contours of the slice sectional objects based on the detected seed points, and (iv) to calculate a volume of the target object based on the contours of the reference sectional object and the slice sectional objects.

## Description

The present application claims priority from Korean Patent Application No. 10-2006-0080015 filed on August 23, 2006, the entire subject matter of which is incorporated herein by reference.

### BACKGROUND

### 1. Field

The present invention generally relates to a system and method for image processing, and more particularly to a system and method for image processing adapted to calculate the volume of a specific portion in a displayed targeted object.

### 2. Background

An image processing system is typically used to display an image of an object of interest. For example, an image processing system for ultrasound diagnosis ("ultrasound system") is widely used in the medical field since it does not invade or destroy a targeted object such as a human internal organ. Recent high-end ultrasound systems are being used to form a 2-dimensional or 3-dimensional image of the internal shape of a targeted object (e.g., human internal organs such as a heart, liver, lung, etc.).

Generally, an ultrasound system has a probe including a wideband transducer for transmitting and receiving ultrasound signals. The transducer is electrically stimulated, thereby generating ultrasound signals and transmitting them into a human body. The ultrasound signals transmitted into the human body are reflected from the boundary of internal organs in the human body. The reflected ultrasound signals, which are forwarded from the boundary of the internal organs in the human body to the transducer, are converted into electrical signals. Then, the converted electrical signals are amplified and signal-processed, thereby generating ultrasound data for the image of the internal organs.

The ultrasound system functions to calculate the volume of a specific portion of the targeted object based on the obtained ultrasound data. First, the ultrasound system displays a 2D ultrasound image of the targeted object corresponding to a reference section. It then receives user information regarding a number of slice sections, which are perpendicular to the reference section, and a seed point on each slice section. Thereafter, the volume of the targeted object can be calculated based on the number of slice sections and the seed point.

In the conventional ultrasound system, however, only one seed point is set to simplify calculation at the center of each slice section, where the location of the seed point can be the same. Accordingly, there have been problems in that the volume of the targeted object cannot be accurately calculated since many errors may occur in finding the contour of the targeted object with only one seed point.

### BRIEF DESCRIPTION OF THE DRAWINGS

Arrangements and embodiments may be described in detail with reference to the following drawings in which like reference numerals refer to like elements and wherein:

FIG. 1 is a block diagram of an ultrasound system constructed in accordance with an embodiment of the present invention.

Fig. 2 is a flow chart showing a procedure of processing an ultrasound image according to an embodiment of the present invention.

Fig. 3 is a flow chart showing a procedure of calculating the volume of a targeted object according to an embodiment of the present invention.

Fig. 4 shows the relationship between a targeted body, a targeted object and a reference section.

Fig. 5 shows how a contour is set on the 2D ultrasound image of a reference section based on contour information inputted by a user according to an embodiment of the present invention.

Fig. 6 shows slice sections and seed points set in the 2D ultrasound image of the reference section shown in Fig. 5 according to an embodiment of the present invention.

Fig. 7 shows how a contour is detected with seed points and their middle point in the 2D ultrasound image of the slice sections shown in Fig. 6 according to an embodiment of the present invention.

### DETAILED DESCRIPTION OF THE PRESENT INVENTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

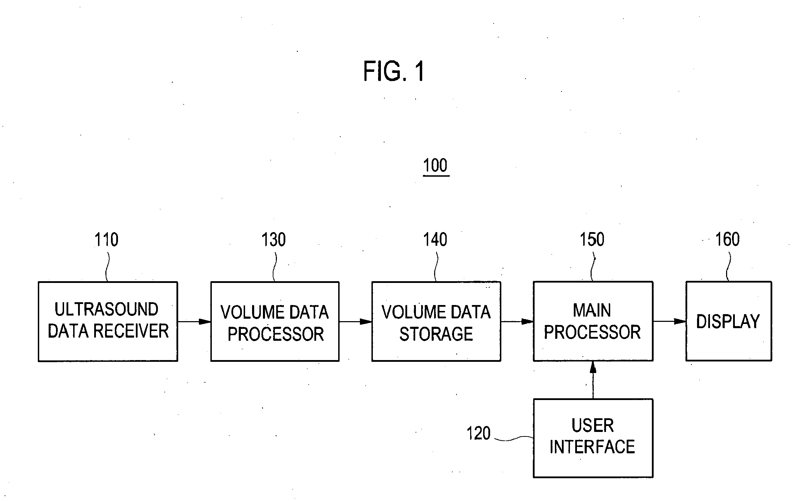

The embodiments of the present invention are described below in view of Figs. 1-7.

Fig. 1 shows an ultrasound system (as an example of an image processing system) constructed in accordance with an embodiment of the present invention. As shown in Fig. 1, an ultrasound system 100 constructed in accordance with the present invention may include an ultrasound data receiver 110, a user interface 120, a volume data processor 130, a volume data storage 140, a main processor 150 and a display 160.

The ultrasound data receiver 110 is configured to transmit an ultrasound signal to a targeted body and receive the reflected ultrasound signal from the targeted body to form an ultrasound image. The ultrasound data receiver 110 is configured to obtain ultrasound data on the targeted body.

Further, the user interface 120 is configured to input reference section information for forming a reference section for a 2D ultrasound image, contour information for manually determining a contour in a 2D ultrasound image, and slice section information for determining slice sections on a 2D ultrasound image. The user interface 120 may include a touchpad, a trackball, a keyboard, etc. Also, the user interface 120 may include a display for inputting information or may be integrated with the display 160. The reference section, the contour and the slice section may be defined as below.

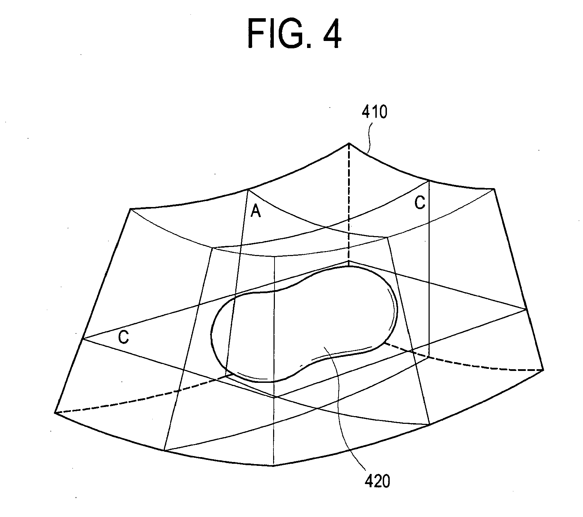

As shown in Fig. 4 illustrating the relationship between a targeted body, a target object and a reference section, the reference section may be section A, section B or section C in volume data formed by the volume data processor 130. The reference section may also be arbitrary sections, which are spaced apart from sections A, B and C. Reference numeral 420 in Fig. 4 indicates a target object, the volume of which is to be calculated, in the targeted body 410.

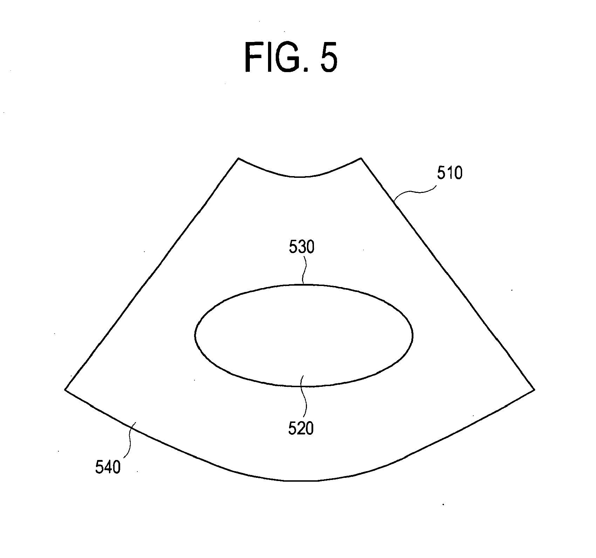

The contour may be used for distinguishing the target object, the volume of which is to be calculated, from other objects in a displayed 2D ultrasound image. For example, as shown in Fig. 5, a contour 530 distinguishes a target object ("reference sectional object") 520 from other objects 540 in a 2D ultrasound image 510.

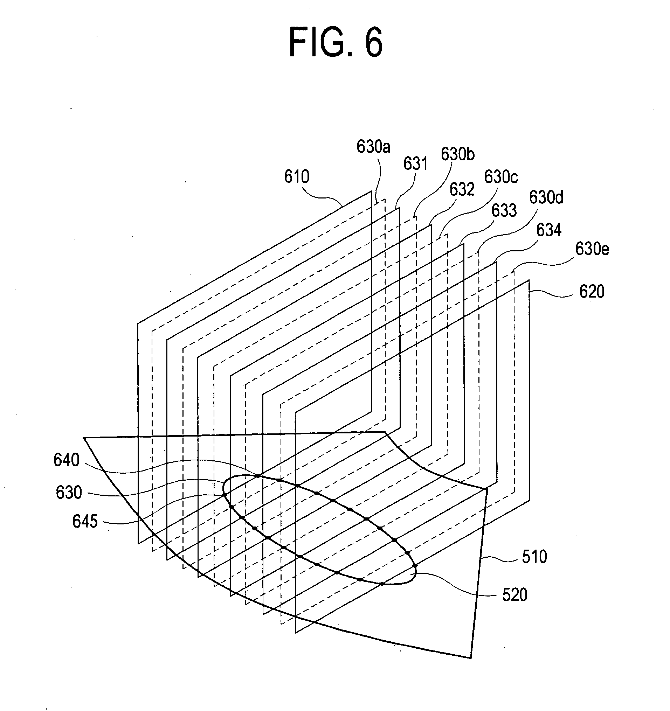

The slice section is a section perpendicular to the reference section. For example, in Fig. 4, when the reference section is section A, the slice section may be sections parallel to section B (including section B) or sections parallel to section C (including section C). The slice section information for determining the slice sections, as shown in Fig. 6, may include the information on reference slice sections 610 and 620 at the two ends of the 2D ultrasound image on the reference section as well as the information on the number of slice sections between the reference slice sections 610 and 620. The locations of reference slice sections and the number of slice sections, which determine the processing time and calculation errors, may be appropriately set by a user based on his/her experience.

As shown in Fig. 4, the volume data processor 130 is configured to receive the ultrasound data on the targeted body 410 from the ultrasound data receiver 110. The volume data processor 130 may further form volume data including the data on the target object 420.

The volume data storage 140 is configured to store the volume data formed by the volume data processor 130. Further, the volume data storage 140 may store predetermined contour information to automatically set the contour of the target object in a 2D ultrasound image. The contour information may be the information, which has been collected in advance, on the forms of various target objects such as internal organs of a human body. The contour of the target object may be set automatically by identifying a similar form with this contour information as the target object in a 2D ultrasound image.

The main processor 150 is configured to extract the ultrasound data corresponding to the reference section based on the reference section information inputted by the user interface 120 and the ultrasound data corresponding to each slice section based on the slice section information inputted by the user interface 120. The main processor 150 may further form 2D ultrasound image signals of the reference section based on the ultrasound data of the reference section. It may also form 2D ultrasound image signals of each slice section based on the ultrasound data of each slice section. The main processor 150 may further set the contour on the 2D ultrasound image of the reference section based on the contour information inputted by the user interface 120. The contour information may be inputted by a user drawing the contour of the target object with a mouse, an electronic pen, etc. directly through the display. Then, the volume of the target object may be calculated based on the contours of the 2D ultrasound image on each slice section and the reference section. The method of determining the contour on the 2D ultrasound image of the slice section will be described later. The main processor 150 may form 3D ultrasound image signals based on the volume data.

The display 160 is configured to receive the 2D ultrasound image signals from the main processor 150 and display a 2D ultrasound image. The display 160 may further display a 3D ultrasound image or a calculated volume value from the main processor 150 when necessary information is inputted to the main processor 150 through the user interface 120.

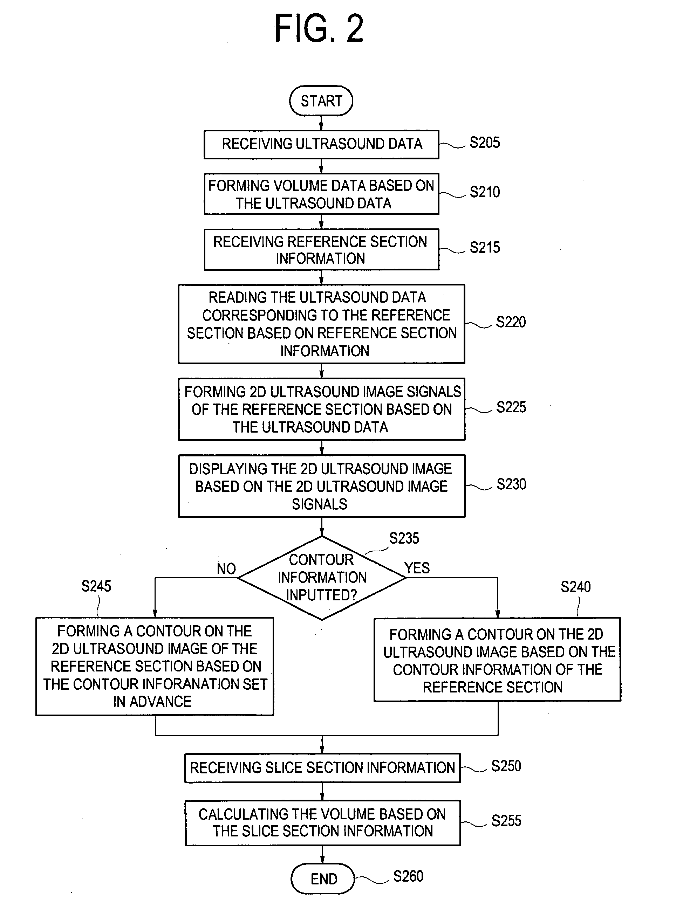

The process of calculating the volume of a target object is now explained in detail with reference to Figs. 2-7.

As shown in Fig. 2, when ultrasound data on a targeted body are obtained through the ultrasound data receiver 110 (S205), the volume data processor 130 may form the volume data of the targeted body based on the ultrasound data (S210). In such a case, the volume data storage 140 may store the volume data formed by the volume data processor 130.

The main processor 150 may receive the information on a reference section through the user interface 120 from a user (S215). It may then read the ultrasound data corresponding to the reference section from the volume data stored in the volume data storage 140 (S220). Further, the main processor 150 may form 2D ultrasound image signals of the reference section based on the read ultrasound data (S225). Then, the display 160 may receive the 2D ultrasound signals from the main processor 150 and display a 2D ultrasound image (S230).

The main processor 150 may determine whether the contour information for setting the contour of the reference sectional object in the 2D ultrasound image through the user interface 120 from the user (S235). If the main processor 150 determines that the contour information is inputted at S235, then it may set the contour 539 of the target object 520 on the 2D ultrasound image 510 based on the inputted contour information, as shown in Fig. 5 (S240). Further, if the main processor 150 determines that the contour information is not inputted at S235, then it may set the contour 530 on the 2D ultrasound image 510 based on the contour information set in advance and stored in the volume data storage 140 (S245).

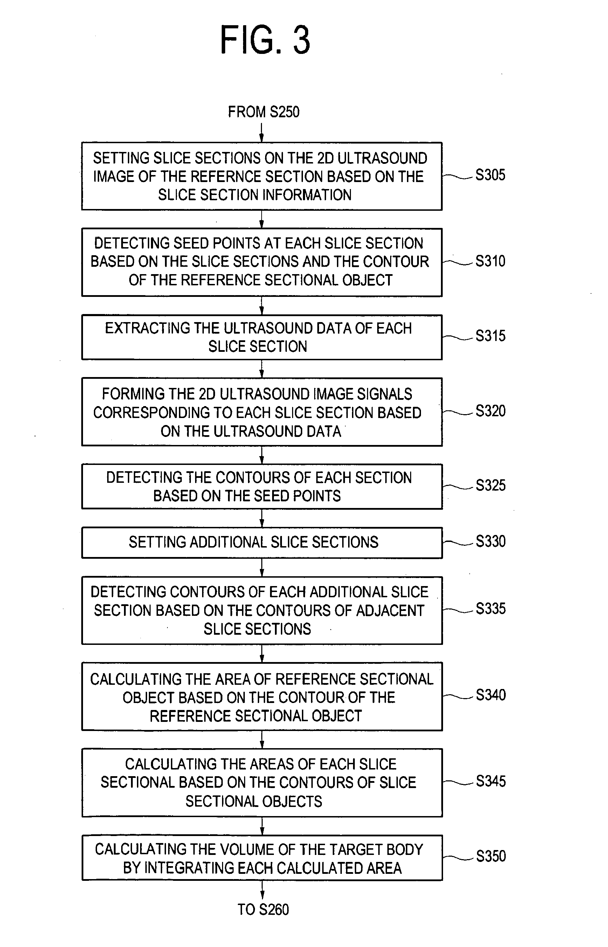

The main processor 150 may receive the slice section information for setting slice sections at the 2D ultrasound image on the reference section through the user interface 120 from the user (S250). The main processor 150 may further calculate the volume of the target object based on the inputted slice section information (S255). S255 will be described later in detail with reference to Figs. 3-7.

Then, the main processor 150 may stop the ultrasound image processing operated in the ultrasound system 100 (S260).

Fig. 3 is a flow chart showing the process of calculating the volume of a target object based on the inputted slice section information according to an embodiment of the present invention.

As illustrated in Fig. 3, after the slice section information is inputted through the user interface 120, the main processor 150 may set slice sections on the 2D ultrasound image of the reference section displayed in the display 160 based on the inputted slice section information (S305). Step S305 is described below in more detail with reference to Fig. 6.

The main processor 150 may be programmed to set the first and second reference slice sections 610 and 620 on the 2D ultrasound image 510 of the reference section based on the reference slice section information of the slice section information.

The main processor 150 may be further programmed to set 4 slice sections 631-634 between the first and second reference slice sections 610 and 620 based on the inputted slice section number information (e.g., the number of slice sections may be 4). In such a case, the slice sections 631-634 may be spaced apart equally between the first and second slice sections 610 and 620.

Also, the main processor 150 may detect seed points for automatically detecting the contour of the target object on each slice section based on the contour of the reference sectional object set in the 2D ultrasound image 510 of the reference section (S310). More specifically, the main processor 150 (shown in Fig. 6) may detect two points, where each slice section meets the contour of the reference sectional object, and set said two points as the seed points 640 and 645.

Further, the main processor 150 may extract ultrasound data corresponding to each slice section from the volume data stored in the volume data storage 140 based on the set slice section (S315). The main processor 150 may further form 2D ultrasound image signals of each slice section based on the extracted ultrasound data (S320). In such a case, the display 160 may receive the 2D ultrasound image signals of each slice section from the main processor 150 and display the 2D ultrasound images of each slice section.

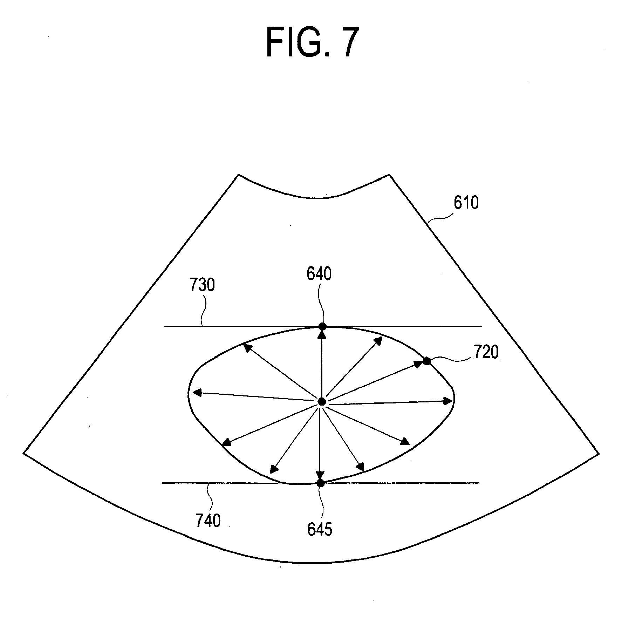

The main processor 150 may further detect the contour of the target object in each slice section ("slice sectional object") based on the seed points of each slice section (S325). The method of how the contour of a slice sectional object is detected is described below with reference to Fig. 7.

The main processor 150 may set a middle point 740 between the two seed points 640 and 645 set on the slice section 610.

The main processor 150 may further detect edges 720, which are spots likely to be the contour of the slice sectional object, with reference to the set middle point 740. In an embodiment of the present invention, the two seed points 640 and 645 may be set and the edges 720 of the slice sectional object may be determined between the ranges 730 and 740 defined by the two seed points 640 and 645. On the other hand, the conventional system caused many errors since it set only one seed point at the center of a slice section. The detailed description of the method for detecting the edges 720 is omitted herein since the conventional methods may be used.

The main processor 150 may further connect the detected edges 720 and form the contour of the slice sectional object.

When the contour of the slice sectional object at every slice section 610, 620, and 631-634 is set through the above procedure, the main processor 150 may set virtual additional slice sections between the two reference slice sections (S330). It may then detect the contour of the slice sectional object at each additional slice section based on the contour of the slice sectional object at the adjacent slice section by using the linear interpolation method (S335). Steps S330 and S335 are described below with reference to Fig. 6.

The main processor 150 may set an additional slice section 630a between the slice sections 610 and 631 (procedure (i)).

The main processor 150 may further detect the contour of the slice sectional object at the additional slice section 630a by applying the linear interpolation method to the contours of the slice sectional object at the slice sections 610 and 631 (procedure (ii)).

The main processor 150 may further set additional slice sections 630b-630e through said procedure (i) and detect the contour of the slice sectional object at each slice section 630b-630e through said procedure (ii).

The main processor 150 may calculate the area of the reference sectional object based on the contour of the reference sectional object (S340) and calculate the areas of each slice sectional object at each slice section 610, 620, 631-634 and 630a-630e (S345). The detailed description of the method for calculating the areas with the contour is omitted herein since the conventional methods may be used.

The main processor 150 may further calculate the volume of the target object by integrating the areas calculated at S340 and S345 (S350). In such a case, the display 160 may receive the calculated volume value from the main processor 150 and display the same.

As described above, according to an embodiment of the present invention, two seed points may be automatically and accurately detected for each slice section based on the contour of the reference sectional object in the 2D ultrasound image of the reference section and the number of slice sections set on the 2D ultrasound image at the reference section. Further, the present invention may also accurately detect the contour of the slice sectional object at each slice section, thereby accurately calculating the volume of the target object.

In accordance with one embodiment of the present invention, there is provided an image processing system, comprising: a volume data receiver to form volume data of a target object based on image data thereof; a user interface to receive reference section information on a reference section, slice section information on a plurality of slice sections each being perpendicular to the reference section, and contour information from a user; a display to form an image of the reference section and images of the slice sections based on the volume data; and a main processor to set a reference sectional object and a slice sectional object based on the reference section information and the slice section information, respectively, said reference sectional object corresponding to the target object in the image of the reference section and said slice sectional object corresponding to the target object in the images of the slice sections, said main processor further being operable to detect a contour of the reference sectional object based on the contour information, to detect seed points of the target object in the images of the slice sections based on the contour information and the slice section information, to detect contours of the slice sectional objects based on the detected seed points, and to calculate a volume of the target object based on the contours of the reference sectional object and the slice sectional objects.

In accordance with another embodiment of the present invention, there is provided an image processing method, comprising: forming volume data of a target object based on image data thereof; receiving reference section information on a reference section from a user; forming an image of the reference section based on the received reference section information; receiving contour information on a contour of a reference sectional object and slice section information on a plurality of slice sections each being perpendicular to the reference section from the user, wherein said reference sectional object corresponding to the target object in the image of the reference section; setting seed points based on the received contour information and the received slice section information; detecting contours of slice sectional objects based on the seed points, said slice sectional object corresponding to the target object in the images of the slice sections; and calculating a volume of the target object based on the contours of the reference sectional object and the slice sectional objects.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An image processing system, comprising
a volume data receiver to form volume data of a target object based on image data thereof;
a user interface to receive reference section information on a reference section of the target object, slice section information on a plurality of slice sections each being perpendicular to the reference section and contour information from a user;
a display to form an image of the reference section and images of the slice sections based on the volume data; and
a main processor to set a reference sectional object and slice sectional objects based on the reference section information and the slice section information, respectively, said reference sectional object corresponding to the target object in the image of the reference section and said slice sectional objects corresponding to the target objects in the images of the slice sections,
said main processor further being operable to detect a contour of the reference sectional object based on the contour information, to detect seed points of the target objects in the images of the slice sections based on the contour information and the slice section information, to detect contours of the slice sectional objects based on the detected seed points, and to calculate a volume of the target object based on the contours of the reference sectional object and the slice sectional objects.

2. The image processing system of Claim 1, wherein the image data is ultrasound data.

3. The image processing system of Claim 1, further comprising a volume data storage to store the volume data.

4. The image processing system of Claim 1, wherein the main processor is further operable to:
set a plurality of the slice sections at the image of the reference section for obtaining images of the slice sections based on the slice section information;
detect points wherein the contour of the reference sectional object and each slice section meets and set said points as seed points of each slice section; and
detect the contours of the slice sectional objects in each slice section based on the seed points.

5. The image processing system of Claim 4, wherein the main processor is further operable to:
detect a middle point between two seed points set in each slice section;
detect edges of the slice sectional object radially from the middle point; and
connect the detected edges to thereby form the contour of the slice sectional object.

6. The image processing system of Claim 5, wherein the main processor is further operable to:
calculate an area of the reference sectional object based on the contour of the reference sectional object;
calculate an area of each slice sectional object on each slice section based on the contours of the slice sectional objects; and
calculate the volume of the target object based on the areas of the reference sectional object and the slice sectional objects.

7. A method of implementing image processing, comprising:
(a) forming volume data of a target object based on image data thereof;
(b) receiving reference section information on a reference section of the target object from a user;
(c) forming an image of the reference section based on the received reference section information;
(d) receiving contour information on a contour of a reference sectional object and slice section information on a plurality of slice sections each being perpendicular to the reference section from the user, wherein said reference sectional object corresponds to the target object in the image of the reference section;
(e) setting a contour of the reference sectional object in the image of the reference section based on the received contour information;
(f) forming images of the slice sections based on the received slice section information;
(g) setting seed points based on the contour of the reference sectional object and the slice sections;
(h) detecting contours of slice sectional objects based on the seed points, said slice sectional objects corresponding to the target objects in the images of the slice sections; and
(i) calculating a volume of the target object based on the contours of the reference sectional object and the slice sectional objects.

8. The method of Claim 7, wherein the image data is ultrasound data.

9. The method of Claim 7, wherein step (g) comprises:
(g1) detecting points wherein the contour of the reference sectional object and the slice sections meet; and
(g2) setting the detected points as the seed points.

10. The method of Claim 7, wherein step (h) comprises:
(h1) detecting a middle point between two seed points set in each slice section; and
(h2) detecting edges of the slice sectional object radially from the middle point.

11. The method of Claim 10, wherein step (i) comprises:
(i1) calculating an area of the reference sectional object based on the contour of the reference sectional object;
(i2) calculating an area of each slice sectional object in each slice section based on the contours of the slice sectional objects; and
(i3) calculating the volume of the target object based on the areas of the reference sectional object and the slice sectional objects.
